# EUROPEAN PATENT APPLICATION

(11) **EP 2 382 998 A1**
(43) Date of publication of application: **02.11.2011**
(21) Application number: 10161162.2
(22) Date of filing: 27.04.2010
(51) Int. Cl.: A61L 27/30, A61L 27/42

(54) **A joint prosthesis**

(71) Applicant: Sandvik Intellectual Property AB, 811 81 Sandviken (SE)
(72) Inventor: Östhols, Erik, SE 141 31, Huddinge (SE); Brandt, Gunnar, SE-170 63, SOLNA (SE); Strömberg, Daniel, 11238 Stockholm (SE); Shen, Zhijian, 16972 Solna (SE)

(57) **Abstract**

A joint prosthesis comprises a first component having a bearing surface and a second component having a bearing surface arranged to articulate with the bearing surface of the first component, said bearing surfaces of the first and the second component thus forming a bearing couple. The bearing surfaces consist of a silicon carbide whisker reinforced alumina. Thus, a bearing couple with low wear, low friction and high hardness is provided.

## Description

The present disclosure relates in general to a joint prosthesis comprising a first component having a bearing surface and a second component having a bearing surface arranged to articulate with the bearing surface of the first component, said bearing surfaces of the first and second components thus forming a bearing couple, and wherein the bearing surface of the first component consists of an alumina based ceramic material.

### BACKGROUND

Orthopedic joint prostheses are known for replacement of joints such as hips, knees, ankles, shoulders, toes or the like. In such prostheses, the surfaces that articulate against each other, called a bearing couple, can be combined from different materials to best suit a patient's needs. In hip prostheses for example, a bearing couple comprises a ball, mounted on the femoral stem, which articulates in a cup mounted in the hipbone. The predominant materials used for hip prostheses today are Co-Cr-alloys for the ball and Ultra High Molecular Weight Polyethylene (UHMWPE) for the cup. However, polyethylene cups have had problems with release of wear particles, which may cause osteolysis.

To decrease wear rates, metal-on-metal bearings can be used, usually of Co-Cr-Mo alloys. A major concern with metal-on-metal bearings is the release of ionic debris. Elevated levels of metals have been seen in patients' serum and urine. Therefore, possible systematic toxicity and cancer risks are considered to constitute disadvantages. Titanium, which is another possibility due to its biocompability, is generally too soft to be useful in femoral head applications.

In order to minimize wear of the bearing couple, ceramics are sometimes used. For example, it has been shown that alumina articulating on alumina experiences the least amount of wear. The most frequently used ceramics are alumina (Al₂O₃) and zirconia (ZrO₂) or mixtures thereof. Examples of orthopedic prostheses of these materials are disclosed for example in US 6,387,132 and WO 01 /17464. Silicon nitride based ceramics have also been proposed, which is disclosed in for example WO 99/47471 and US 6,881,229.

The greatest benefit of the ceramic materials is the low wear rate compared to both UHMWPE and metals. However, a major concern is the brittle nature of ceramics, especially alumina, and the risk for impingement, which is one of the common reasons for fracture.

In order to reduce the risk of brittle failure of alumina, it has been proposed to reduce the grain size to increase the bending strength. It has also been proposed to add zirconia grains to increase the toughness. Such ceramics are commonly denominated zirconia toughened alumina (ZTA).

Pure zirconia has been used for bearing surfaces as mentioned above, but sometimes with disastrous results. Zirconia has a very high toughness compared to other ceramic materials, but only moderate hardness. Because of poor thermal conductivity and the metastable nature of the often yttria stabilized tetragonal and/or cubic phases used in industrial zirconia, phase transformations to the thermodynamically stable (at low temperature) monoclinic phase may take place upon articulation. The accompanying volume change may lead to fracture. This problem may also be present, to a lesser degree, in ZTA, though the problems can be minimized with careful processing of the ceramic material.

Burger et al. "High Strength and Toughness Alumina Matrix Composites by Transformation Toughening and 'In Situ' Platelet Reinforcement (ZPTA) - The New Generation of Bioceramics", Key Engineering Materials Vols. 192-195 (2001) pp. 545-548, discusses various materials for use in biomedical applications, such as ball heads and cups for total hip prostheses systems. A zirconia and platelet reinforced alumina ceramic (ZPTA) is proposed due to its high strength and low wear rate. Burger et al. also mention that alumina may be toughened by distribution of silicon carbide whiskers in order to cause crack deflection, but state that carcinogenicity of silicon carbide whiskers and limited adhesion between the matrix and whiskers within the microstructure have limited the exploitation. In addition, Burger et al. describe additions of chromia to increase hardness as a compensation for the decreased hardness when adding stabilized zirconia. Chromium, however, has known issues with regard to toxicity.

It is thus clear that there is still a need for a suitable material for the bearing surfaces of a bearing couple. Such a material has to have high wear resistance and low friction. It also has to have sufficient toughness in order to avoid the risk of brittle failure. Furthermore, it has to be chemically stable so as to not cause any health issues when inserted into a patient.

### SUMMARY

The object of the invention is to provide a joint prosthesis wherein the bearing surfaces of the bearing couple exhibit low wear, have low friction and sufficient toughness. Moreover, the bearing couple should be chemically stable.

The object is achieved by means of the joint prosthesis in accordance with claim 1. Embodiments are defined by the dependent claims.

The object is also achieved by use of an alumina based ceramic material comprising 2-60 % by weight silicon carbide whiskers distributed in the alumina matrix, optionally up to 20 % by weight of zirconia and optionally up to 30 % by weight of cubic carbides, nitrides and/or carbonitrides as a bearing surface in a joint prosthesis.

The joint prosthesis according to the present invention comprises a first component having a bearing surface and a second component having a bearing surface arranged to articulate with the bearing surface of the first component, said bearing surfaces of the first and the second component thus forming a bearing couple. The bearing surface of the first component consist of an alumina based ceramic material comprising 2-60 % by weight of silicon carbide whiskers distributed in the alumina matrix, optionally up to 30 % by weight of cubic carbides, nitrides and/or carbonitrides, and optionally up to 20 % by weight of zirconia. The alumina based ceramic material comprising the silicon carbide whiskers has high toughness, high wear resistance, low friction, high hardness, high Weibull modulus and high chemical stability.

Preferably, also the bearing surface of the second component consists of an alumina based ceramic material comprising 2-60 % by weight of silicon carbide whiskers distributed in the alumina matrix, optionally up to 30 % by weight of cubic carbides, nitrides and/or carbonitrides, and optionally up to 20 % by weight of zirconia. This will result in a ceramic-on-ceramic bearing couple *inter alia* with very low wear rate and high toughness.

The alumina based ceramic material may optionally also comprise grain growth inhibiting and strengthening additions, such as magnesia and/or yttria, up to 1 % by weight of each, preferably up to 1 % by weight in total. Such additions reduce the risk of significant grain growth during sintering of the material, as well as improve the mechanical and chemical properties of the intergranular phase, and are thus beneficial for the strength of the material.

In accordance with one embodiment, the alumina based ceramic material comprises 10-45 % by weight of silicon carbide whiskers, preferably 15-40 % by weight of silicon carbide whiskers.

The alumina may suitably have an average grain size of equal to or less than 2 µm, preferably equal to or less than 1 µm, after sintering. A small grain size is desirable *inter alia* since it increases the bending strength of the material.

The silicon carbide whiskers may suitably have an aspect ratio of 5-100, preferably 5-50, most preferably 5-25. Moreover, the diameter of the silicon carbide whiskers is suitably maximally 2 µm, preferably maximally 1 µm. In accordance with one embodiment the alumina based ceramic material comprises at least 40 % by weight of alumina, preferably at least 50 % by weight of alumina. In accordance with another embodiment, the silicon carbide whiskers are whiskers synthesized by pyrolysis of rice hulls.

In accordance with yet another embodiment, the alumina based ceramic material comprises 2-30 % by weight, preferably 3-20 % be weight, of cubic carbides, nitrides and/or carbonitrides. The carbides, nitrides and/or carbonitrides are present as small particles, suitably with a grain size of less than or equal to 5 micrometer, preferably less than or equal to 3 micrometer and may thus reinforce the material.

The process for producing the joint prosthesis comprises producing the alumina based ceramic material comprising 2-60 % by weight silicon carbide whiskers distributed in the alumina matrix, optionally up to 30 % by weight of cubic carbides, nitrides and/or carbonitrides, and optionally up to 20 % by weight of zirconia by mixing the raw materials of the different components of the alumina based ceramic material in a dispersion, drying said dispersion to a powder, compacting and sintering the obtained powder to obtain a near-net-shape of the bearing surface of the first component.

Preferably, drying of the dispersion is performed by freeze granulation. Freeze granulation results in better wear properties of the material after sintering compared to spray drying. As mentioned above, the silicon carbide whiskers are preferably produced by pyrolysis of rice hulls.

According to one preferred embodiment, sintering is performed by hot isostatic pressing since this gives a more random orientation of the whiskers in the material compared to other possible sintering methods of the material. Preferably, cold isostatic pressing is performed prior to the hot isostatic pressing as this gives an even more pronounced random orientation of the whiskers in the sintered material.

### DETAILED DESCRIPTION

In the present disclosure, alumina based ceramic material shall be considered to mean a material comprising an alumina grain matrix. When the material comprises zirconia, the alumina based material shall be considered to mean a material comprising an alumina grain matrix further comprising zirconia. Thus, it should be noted that the term "alumina based" does not automatically mean that alumina is the major constituent of the material. However, the alumina based ceramic material should preferably comprise at least 40 % by weight of alumina, more preferably at least 50 % alumina.

The joint prosthesis in accordance with the present invention comprises a first component having a bearing surface and a second component having a bearing surface arranged to articulate with the bearing surface of the first component, said bearing surfaces of the first and the second component thus forming a bearing couple. At least the bearing surface of the first component consists of an alumina based ceramic material reinforced with silicon carbide whiskers.

Silicon carbide whisker reinforced alumina is a previously known material used in cutting tools inserts. Such cutting tool inserts are an established product on the cutting tool marked, mainly for machining of heat resistant materials, and to some extent also for machining of cast iron.

One example of an alumina based ceramic material comprising silicon carbide whiskers is described in US 4,543,345 which discloses a dense, sintered material comprised of an alumina grain matrix, with an addition of 4-55 % by weight of silicon carbide whiskers. The silicon carbide whiskers have a length of 10-80 µm and a diameter of 0.6 µm. Moreover, the silicon carbide whiskers are single crystals of alpha or beta silicon carbide.

Another example may be found in US 4,789,277 which discloses the use of this type of material for cutting tools. It is disclosed that silicon carbide whisker contents of 1.6-35 % by weight give the best mechanical properties (toughness, bending strength), as well as performance in cutting, with a preferred range of 16-30 % by weight of whiskers, and 20 % by weight giving especially good performance.

Furthermore, US 5,449,647 discloses a silicon carbide whisker reinforced alumina material with 25 % by weight of whiskers for cutting tools, wherein the average whisker length is 4-7 µm, with 95 % of the whiskers being less than 10 µm in length.

Even though silicon carbide whisker reinforced alumina as such is known for use in cutting tools, this type of material is not frequently used for other applications. This is probably due to its complexity. However, in accordance with the present invention, this type of material is used in a joint prosthesis. It has been found to be an excellent choice of material for such an application.

As mentioned above, at least one of the bearing surfaces of the bearing couple consists of an alumina based ceramic material in accordance with the present invention. The alumina based ceramic material comprises silicon carbide whiskers homogeneously distributed in the material, which reinforces the alumina. The silicon carbide whiskers are arranged between the grains of the alumina and toughen the material. Moreover, the whiskers reduce the apparent defect size of the material, an effect which manifests itself in significantly higher fracture toughness (K1c), modulus of rupture value and Weibull modulus than for pure alumina or zirconia reinforced alumina without whisker addition. The effect is at least partly due to the difference in heat expansion coefficient between silicon carbide and alumina, which results in the silicon carbide whiskers experiencing significant pressure in the alumina matrix after sintering, and also weakening of the chemical bonds between silicon carbide whiskers and alumina matrix during the thermal movement of the grains during cooling.

Although other alumina based materials, such as ZTA with platelet additions, can be brought to high values of K1 c, MOR and Weibull modulus, this requires very careful processing with clean room technology, and even then there is always a risk that defects introduced through instances of abnormal grain growth and/or contaminations will increase the brittleness of the material. With the silicon carbide whiskers addition, the adverse effects of single large grains, inclusions etc. are effectively masked by the whisker reinforcement, making the material much less dependent on difficult and expensive processing technology, such as clean room processing.

Alumina in itself has low friction, especially against itself, and the desired hardness for use as a bearing surface. Moreover, the silicon carbide whisker reinforced alumina is chemically stable under the conditions found in biomedical applications. Therefore, the silicon carbide whisker reinforced alumina based ceramic material is highly reliable and may be expected to have a long service life in a joint prosthesis.

The alumina based ceramic material may optionally also comprise zirconia (ZrO₂) as well as cubic carbides, nitrides and/or carbonitrides. Moreover, the ceramic material may optionally comprise grain growth inhibiting and sintering additives, such as yttria (Y₂O₃) and magnesia (MgO), which may also strengthen the material.

In Table 1, silicon carbide whisker reinforced alumina is compared to other known ceramic materials used in bearing couples. The typical values of the elastic modulus, hardness, K1 c and strength are shown for the different materials. The given values are approximate, as they depend on processing parameters and raw materials used, and should only be understood as a rough guide. As discussed above, though it has very high toughness, pure zirconia suffers from problems with phase stability. Silicon nitride, which has very high toughness, is subject to oxidation and thus degrades in contact with moist air and/or water, although slowly. Also, because whisker reinforced alumina has excellent toughness and reliability even without zirconia additions, its hardness exceeds that of ZTA materials used in implant applications today. This also makes additions of potentially toxic chromia unnecessary.

**Table 1**

| Material | Elastic modulus [GPa] | Hardness [GPa] | K1c [MPam^{½}] | Strength [MPa] |
|---|---|---|---|---|
| Alumina | 380-400 | 17-19 | 3-4 | 300-400 |
| Zirconia | 200-220 | 12-14 | 10-12 | 1000-1200 |
| Si₃N₄ | 300-320 | 14-16 | 8-10 | 950-1200 |
| ZTA | 340-360 | 16-17 | 5-7 | 850-1150 |
| Al₂O₃-SiC_{whisker} | 350-450 | 20-21 | 5-7 | 900-1100 |

The silicon carbide whiskers are single crystals of silicon carbide. The whiskers are shaped like needles, and have a very small diameter and high aspect ratio. The most significant property of the silicon carbide whiskers is their very high strength approaching the theoretical strength of SiC. This is due to the very small diameter and the crystalline perfection.

Silicon carbide whiskers used to reinforce the alumina are of a very small diameter and high aspect ratio, as mentioned above. Like other fibrous materials, such as asbestos, the silicon carbide whiskers have been found to cause health problems when inhaled. Moreover, it has been found that the geometry, i.e. diameter and length, is the decisive factor *inter alia* because the time fibrous material will stay in the breathing zone before settling out is highly dependent on the diameter. However, if fibers like mineral-asbestos and other difficult-to-dissolve fibers have a length smaller than 3 µm, the fibers have no cytotoxic effect.

If the surface of a component of the bearing couple for some reason would be worn out, small silicon carbide particles could possibly be released from the material. Longer whiskers incorporated in the material would break during wear before being released, partly because of the pressure exerted on them by the surrounding alumina grains, as discussed above. Small silicon carbide particles do however not pose any health risk and are essentially chemically stable in the human body. Hence, there is no health risk related to the silicon carbide whiskers when the material is used in the components of the bearing couple.

As noted above, the presence of the silicon carbide whiskers in the alumina based ceramic material increases the bending strength and the fracture toughness of the alumina and hence the reliability of the material. It also increases the thermal conductivity and the thermal shock resistance. Considering also the high hardness and low wear and friction, and the high Weibull modulus, the silicon carbide whisker reinforced alumina based material is an excellent candidate material for a bearing surface of a joint prosthesis.

The alumina based ceramic material comprises 2-60 % by weight of silicon carbide whiskers. If the silicon carbide whiskers are present in an amount less than 2 % by weight, the effect of the whiskers are generally insufficient to achieve the desired fracture toughness of the material. However, if present in an amount above 60 % by weight, the bending strength of the material decreases. Preferably, the silicon carbides whiskers are added in an amount of at least 10 % by weight, more preferably at least 15 % by weight. Moreover, the silicon carbide whiskers are preferably not present in more than 45 % by weight, more preferably up to 40 % by weight. By way of example only, the material could for example comprise about 20-30 % by weight of silicon carbide whiskers.

The silicon carbide whiskers suitably have an aspect ratio of 5-100. The aspect ratio is the ratio between the length and the diameter of the whiskers. Preferably, the silicon carbide whiskers have an aspect ratio of 5-50, more preferably 5-25.

Moreover, the silicon carbide whiskers suitably have a diameter of maximally 2 µm. Whiskers with a larger diameter have lower strength due to inclusion of more crystal defects, and would also act as defects in the alumina based ceramic material and should therefore be avoided. The whiskers normally have a diameter of at least 0.1 µm. In accordance with a preferred embodiment, the diameter of the silicon carbide whiskers is maximally 1 µm, preferably 0.3-0.8 µm.

The length of the silicon carbide whiskers when present in the dense alumina based ceramic should preferably be 1-100 µm. The length of the whiskers in the final product depends on the process for producing the material as the mixing and granulation of the raw materials before forming the green body inevitably reduces the length of the whiskers.

Furthermore, when in the form of a raw material, the silicon carbide whiskers may have free carbon, such as up to 0.5 % by weight of the whiskers, on the outer surface thereof as a result of the synthesizing process. The carbon may decrease the oxygen content of the surface of the whiskers, thereby improving the properties of the final material.

Silicon carbide whiskers can for example be produced by pyrolysis of rice hulls, chemical vapour deposition and carbo-thermal reduction of silica. However, synthesis of silicon carbide whiskers by pyrolysis of rice hulls has shown to provide the best results. It has been found that the alumina based ceramic material has better mechanical properties in case the whiskers are produced by means of pyrolysis from rice hulls compared to the other above mentioned production methods. The synthesis of silicon carbide whiskers from rice hulls is as such previously known and originates from a process disclosed in US 3,754,076 which is hereby incorporated by reference.

The alumina raw material may suitably be alpha-alumina. Moreover, the average grain size of the alumina after sintering should not be too large since this lowers the modulus of rupture and reduces reliability. Furthermore, a small grain size after sintering is believed to be advantageous for the wear resistance. Therefore, the average grain size of the alumina in the dense alumina based ceramic material is suitably equal to or less than 2 µm, preferably equal to or less than 1 µm.

Naturally, the alumina raw material powder may be produced by any conventional technique resulting in a powder with a suitable grain size. Manufacturing processes for alumina powders are as such previously known and will therefore not be described in the present disclosure.

In accordance with one embodiment, the alumina based ceramic material may comprise up to 30 % by weight of cubic carbides, nitrides and/or carbonitrides. Examples of such cubic carbides, nitrides and/or carbonitrides are TiC, TaC, Ti(C,N), TiN, TaN or the like. The purpose of the addition of cubic carbides, nitrides and/or carbonitrides is to increase the strength of the material. The carbides, nitrides and/or carbonitrides are present as small particles, and may suitably have a grain size of less than or equal to 5 µm, preferably less than or equal to 3 µm.

Preferably, the cubic carbides, nitrides and/or carbonitrides may be added such that they are present in an amount of 2-30 % by weight, more preferably 3-20 % by weight, in the material. This may also enable a reduced content of silicon carbide whiskers if desired, while still achieving the desired properties, such as strength of the material. For example, if at least 2 % of cubic carbides, nitrides and/or carbonitrides are added, the content of silicon carbide whiskers may preferably be maximally 45 % by weight, more preferably maximally 40 % by weight. The cubic carbides, nitrides and/or carbonitrides may be produced by conventional techniques.

In accordance with yet another embodiment, the alumina based ceramic material may comprise up to 20 % by weight of zirconia, preferably up to 15 % by weight of zirconia. The purpose of zirconia is to improve the toughness of the material in the same way as in the case of ZTA. If zirconia is added, it should preferably be present in an amount of at least 1 % by weight in order to get the desired effect, more preferably at least 2 % by weight.

The zirconia raw material may be produced by conventional techniques to achieve a suitable raw material powder. If desired, the zirconia raw material may constitute partially stabilized zirconia (PSZ) without departing from the scope of the invention. However, it is not necessary to utilize partially stabilized zirconia as raw material in order to achieve the desired effect.

The alumina based ceramic material may also comprise conventional grain growth inhibiting and sintering additions, such as yttria and/or magnesia, in order to reduce the risk of pronounced grain growth during sintering and increase the strength of the material. For example, the alumina based ceramic may comprise up to 1 % by weight of yttria and/or up to 1 % by weight of magnesia, preferably up to 1 % by weight in total. According to one embodiment, the alumina based ceramic material comprises 0.01-0.1 % by weight of yttria. According to another embodiment, the alumina based ceramic material comprises 0.01-0.1 % by weight of magnesia.

The alumina based ceramic material used in a bearing surface of the bearing couple in accordance with the present invention may thus comprise:
2 - 60 % by weight of silicon carbide whiskers
0 - 20 % by weight of ZrO₂
0 - 1 % by weight of Y₂O₃
0 - 1 % by weight of MgO
0 - 30 % by weight of cubic carbides, nitrides and/or carbonitrides
balance Al₂O₃ and normally occurring impurities resulting from the manufacturing process.

The silicon carbide whisker reinforced alumina material used in accordance with the present invention may be produced by firstly mixing the raw materials of the various components to a raw material powder mixture. In order for the powder mixture to have a proper flowability to be suitable for compaction, the powder mixture has to be granulated. The most common way of granulating such a powder mixture is by spray drying. Spray drying means that a slurry containing the powdery components of the final composition is sprayed into a stream of hot gas, creating dry granules of powder mixture. However, during spray drying, the whiskers are usually concentrated in the middle of the granules and the granules borders are generally depleted of whiskers. This leads to an inhomogeneous structure of the sintered material.

Another alternative to granulate the power mixture is freeze granulation. Freeze granulation leads to a more homogenous distribution of the whiskers of the compacted material since it results in granules with a homogenous distribution of whiskers in the matrix. Freeze granulation comprises thoroughly mixing the raw materials in a dispersing medium. Suitable binders may optionally be added to the dispersion. The dispersion is thereafter sprayed into a vessel having a temperature well below the freezing point of the dispersing medium, preferably into a vessel containing liquid nitrogen. Thereby, frozen granules with essentially the same morphology as the dispersion are obtained. The frozen granules are then transferred into a freeze drier where the frozen liquid is sublimated at a suitable pressure and temperature.

It has been found that the silicon carbide whisker reinforced alumina has approximately half the wear rate when freeze granulation has been used, compared to when spray drying has been used, when testing using an alumina abrasive slurry as well as a silicon carbide abrasive slurry. In the material made from spray dried powder, entire agglomerates were sometimes released due to preferential wear of agglomerated alumina grains. This did not happen in the material made from freeze dried powder. Therefore, freeze granulation is the preferred granulation process.

The powder obtained from the granulation is then compacted and sintered by conventional techniques, such as by hot pressing or hot isostatic pressing. It is also possible to utilize spark plasma sintering. Preferably, hot isostatic pressing is used as this sintering process enables a more random orientation of the silicon carbide whiskers in the material compared to hot pressing or spark plasma sintering. Preferably, cold isostatic pressing is performed prior to the hot isostatic pressing as this gives an even more pronounced random orientation of the whiskers in the sintered material.

Sintering is preferably performed to a density of the silicon carbide whisker reinforced alumina of at least 3.5 g/cm³, more preferably to equal to or more than 3.7 g/cm³.

The surface of the sintered silicon carbide whisker reinforced alumina based ceramic may thereafter be ground and optionally polished in accordance with previously known techniques in order to obtain a suitable surface finish for the bearing surface.

It will be readily apparent to the skilled person that possible binders and sintering additives may be added during the production of the silicon carbide whisker reinforce alumina based ceramic material in accordance with conventional processes for producing such a material without departing from the scope of the invention.

As previously mentioned, the silicon carbide whisker reinforced alumina based material has a high toughness and hardness. By way of example only, for a silicon carbide whisker reinforced alumina comprising about 20-30 % by weight of whiskers with a diameter of about 0.4-0.7 µm and a length of about 1-10 µm, having an average grain size of the alumina of about 1.1-1.3 µm and a density of about 3.74 g/cm³, a hardness Hv10 of about 2030 kg/mm² and a K1 c of about 7 Mpam^{½} have been determined. Moreover, such a material has a Weibull modulus of at least 13.

Further examples of compositions of the alumina based ceramic materials, and their hardness (Hv10) and fracture toughness (K1 c) at an obtained density are specified in Tables 2 and 3, respectively. Sintering was performed by hot pressing for Examples A-H and by cold isostatic pressing followed by hot isostatic pressing for Example I.

**Table 2**

| Example | SiC_{whisker} [wt%] | ZrO₂ [wt%] | TiN [wt%] | TiC [wt%] | Ti(C,N) [wt%] | MgO [wt%] | Alumina [wt%] |
|---|---|---|---|---|---|---|---|
| A | 16.2 | 7.8 | - | - | - | - | balance |
| B | 28.4 | 8 | - | - | - | - | balance |
| C | 21.6 | 15.3 | - | - | - | - | balance |
| D | 19.4 | 16.3 | - | 2.6 | - | - | balance |
| E | 19.4 | 16.3 | 2.9 | - | - | - | balance |
| F | 19.3 | 16.2 | - | - | 4.8 | - | balance |
| G | 25 | 15 | 6.7 | - | - | 0.05 | balance |
| H | 25 | - | 7 | - | - | 0.05 | balance |
| I | 25 | - | - | - | - | 0.05 | balance |

**Table 3**

| Example | Hv10 [kg/mm²] | K1c [MPam^{½}] | Density [g/cm³] |
|---|---|---|---|
| A | 1900 | 4.3 | 3.9 |
| B | 2000 | 4.8 | 3.8 |
| C | 1850 | 4.5 | 4.0 |
| D | 1810 | Not tested | 4.0 |
| E | 1743 | Not tested | 4.0 |
| F | 1853 | Not tested | 4.0 |
| G | 1950 | 5.7 | 4.0 |
| H | 2150 | 4.2 | 3.8 |
| I | 2062 | 5.8 | 3.7 |

Even though the invention is merely limited to a bearing surface of the bearing couple consisting of the above described alumina based ceramic material, it will be readily apparent to the skilled person that for most cases it is appropriate to produce the entire component of the alumina based ceramic. For example in the case of a hip joint, the entire ball head and/or cup may be formed of the alumina based ceramic material.

Moreover, even though it is preferred that both bearing surfaces are made of the alumina based ceramic material comprising the silicon carbide whiskers, it is also conceivable that one of the bearing surfaces of the bearing couple consists of the aforementioned material whereas the bearing surface of the second component consists of another material, such as alumina, zirconia, ZTA, PZTA, UHMWPE, or a metallic material. If so, it is mostly preferred that the bearing surface of the second component is constituted of a ceramic material, most preferably an alumina based ceramic material, in order to achieve the lowest wear rates.

The joint prosthesis is preferably a hip prosthesis wherein the components of the prosthesis comprise the ball head and the cup. However, the joint prosthesis may also be a knee prosthesis, a shoulder prosthesis or any other kind of joint prosthesis.

## Claims

1. A joint prosthesis comprising a first component having a bearing surface and a second component having a bearing surface arranged to articulate with the bearing surface of the first component, said bearing surfaces of the first and the second component thus forming a bearing couple, **characterized in that** the bearing surface of the first component consists of an alumina based ceramic material comprising 2-60 % by weight of silicon carbide whiskers distributed in the alumina matrix, optionally up to 30 % by weight of cubic carbides, nitrides and/or carbonitrides, and optionally up to 20 % by weight of zirconia.

2. The joint prosthesis according to claim 1 wherein the bearing surface of the second component consists of an alumina based ceramic material comprising 2-60 % by weight of silicon carbide whiskers distributed in the alumina matrix, optionally up to 30 % by weight of cubic carbides, nitrides and/or carbonitrides, and optionally up to 20 % by weight of zirconia.

3. The joint prosthesis according to any of claims 1 or 2 wherein the alumina based ceramic material further comprises grain growth inhibiting and strengthening additions, such as magnesia and/or yttria, up to 1 % by weight of each.

4. The joint prosthesis according to claim 3 wherein the alumina based ceramic material comprises grain growth inhibiting and strengthening additions up to 1 % by weight in total.

5. The joint prosthesis according to any of the preceding claims wherein the alumina based ceramic material comprises 10-45 % by weight of silicon carbide whiskers.

6. The joint prosthesis according to any of the preceding claims wherein the alumina has an average grain size of equal to or less than 2 µm, preferably equal to or less than 1 µm.

7. The joint prosthesis according to any of the preceding claims wherein the whiskers have an aspect ratio of 5-100.

8. The joint prosthesis according to any of the preceding claims, wherein the silicon carbide whiskers have a diameter of maximally 2 µm.

9. The joint prosthesis according to any of the preceding claims wherein the alumina based ceramic material comprises at least 40 % by weight of alumina.

10. The joint prosthesis according to any of the preceding claims wherein the silicon carbide whiskers are whiskers synthesized by pyrolysis of rice hulls.

11. The joint prosthesis according to any of the preceding claims wherein the alumina based ceramic material comprises 2-30 % by weight of cubic carbides, nitrides and/or carbonitrides.

12. Process for producing a joint prosthesis according to any of the preceding claims comprising producing the alumina based ceramic material comprising 2-60 % by weight silicon carbide whiskers distributed in the alumina matrix, optionally up to 30 % by weight of cubic carbides, nitrides and/or carbonitrides, and optionally up to 20 % by weight of zirconia by mixing the raw materials of the different components of the alumina based ceramic material in a dispersion, drying said dispersion to a powder, compacting and sintering the obtained powder to obtain a near-net-shape of the bearing surface of the first component.

13. Process according to claim 12, wherein drying of the dispersion is performed by freeze granulation.

14. Process according to claim 12 or 13 wherein the silicon carbide whiskers are produced by pyrolysis of rice hulls.

15. Process according to any of claims 12 to 14 wherein the sintering is performed by hot isostatic pressing.

16. Process according to claim 15 wherein cold isostatic pressing is performed prior to the hot isostatic pressing.

17. Use of an alumina based ceramic material comprising 2-60 % by weight silicon carbide whiskers distributed in the alumina matrix, optionally up to 20 % by weight of zirconia and optionally up to 30 % by weight of cubic carbides, nitrides and/or carbonitrides as a bearing surface in a joint prosthesis.
